# EUROPÄISCHE PATENTSCHRIFT

(11) **EP 3 367 054 B1**
(45) Veröffentlichungstag und Bekanntmachung des Hinweises auf die Patenterteilung: **06.05.2020**
(21) Anmeldenummer: 17158455.0
(22) Anmeldetag: 28.02.2017
(51) Int. Cl.: G01B 11/25, G01N 21/25

(54) **SYSTEM ZUR OPTISCHEN ERFASSUNG VON OBJEKTEN**
DEVICE FOR OPTICALLY RECORDING OBJECTS
SYSTÈME DE DÉTECTION OPTIQUE D'OBJETS

(43) Veröffentlichungstag der Anmeldung: 29.08.2018
(73) Patentinhaber: Phenospex B.V., 6416 SG Heerlen (NL)
(72) Erfinder: Zhokhavets, Uladzimir, 52066 Aachen (DE); Hummel, Grégoire Martin, 6218 Maastricht (NL); Schwartz, Stefan, 52146 Würselen (DE)
(74) Vertreter: dompatent von Kreisler Selting Werner - Partnerschaft von Patent- und Rechtsanwälten mbB

(56) Entgegenhaltungen:
- EP-A1- 2 799 848
- EP-A2- 1 607 041
- DE-A1-102005 014 525
- GB-A- 2 427 913
- US-A- 6 142 629
- US-B1- 9 134 593
- LUCAS BUSEMEYER ET AL: "BreedVision - A Multi-Sensor Platform for Non-Destructive Field-Based Phenotyping in Plant Breeding", SENSORS, Bd. 13, Nr. 3, 27. Februar 2013 (2013-02-27), Seiten 2830-2847, XP055358966, DOI: 10.3390/s130302830

## Beschreibung

Die vorliegende Erfindung betrifft ein System zur optischen Erfassung von Objekten.

Bekannterweise werden Objekte mittels Laser-Triangulation optisch erfasst. Hierbei wird ein Objekt mittels eines Lasers beleuchtet. Aufgrund der Topologie des Objektes wird die Beleuchtung des Lasers verzerrt.

Durch eine Kamera wird ein Bild des beleuchteten Objekts aufgenommen und aus der Verzerrung der Beleuchtung eine Höheninformation über das Objekt extrahiert. Dabei liefert die Laser-Triangulation ausschließlich eine Höheninformation über die Topologie des Objekts. Somit besteht bei bekannten Laser-Triangulationsverfahren und -systemen lediglich die Möglichkeit das Objekt anhand seiner Höheninformationen weiter zu charakterisieren oder von anderen Objekten zu unterscheiden.

In vielen Fällen lassen sich Objekte nicht allein durch ihre Höheninformation charakterisieren oder voneinander unterscheiden. Dies gilt insbesondere falls es sich bei dem Objekt um ähnliche aber nicht exakt gleiche Objekte handelt, wie beispielsweise Autos, Tiere, Pflanzen oder dergleichen.

Insbesondere bei Pflanzen ist es erforderlich zur Charakterisierung eine Aussage über den Krankheitsstatus, den Ernährungsstatus und/oder die Reife der Früchte der Pflanze treffen zu können. Auch ist es erforderlich, beispielsweise im Feld eine Unterscheidung der einzelnen Pflanzen vornehmen zu können, um gezielt nur bestimmte Pflanzen zu erfassen. All dies ist nicht möglich durch die bekannte Lasertriangulation, da diese nur Höheninformationen liefert.

Lucas Busemeyer et al.: "BreedVision - A Multi-Sensor Platform for Non-Destructive Field-Based Phenotyping in Plant Breeding", Sensors 2013, 13, 27. Februar 2013, Seiten 2830 - 2847 offenbart ein System mit einer Vielzahl von Sensoren zur Erfassung von Pflanzen bzw. Pflanzenwachstum.

EP 2 799 848 beschreibt eine Vorrichtung bei der eine Höheninformation eines Objekts sowie eine Oberflächeninformation jeweils optisch erfasst werden mit separaten Mitteln.

DE 10 2005 014 525 beschreibt eine Vorrichtung zur Ermittlung einer Höheninformation eines Objekts, wobei mittels einer Maske ein Muster aus zwei unterschiedlichen Wellenlängen erzeugt wird. Dabei handelt es sich bei dem Muster um ein Streifenmuster.

Aufgabe der vorliegenden Erfindung ist es daher, ein System zur optischen Erfassung von Objekten zu schaffen, mit verbesserter Möglichkeit zur Charakterisierung des Objekts.

Die Aufgabe wird gelöst durch ein System zur optischen Erfassung von Objekten gemäß Anspruch 1, sowie durch das Verfahren des Anspruchs 10.

Das erfindungsgemäße System zur optischen Erfassung von Objekten weist eine Quelle zur Erzeugung von Licht einer ersten Wellenlänge auf. Dabei wird durch das Licht das Objekt beleuchtet und die Beleuchtung durch das Objekt verzerrt. Weiterhin weist das System wenigstens eine Farblichtquelle zur Erzeugung von Farblicht einer zweiten Wellenlänge auf, wobei durch das Farblicht der Farblichtquelle das Objekt beleuchtet wird. Hierbei ist die erste Wellenlänge verschieden von der zweiten Wellenlänge. Von einem ersten Sensor wird das durch Licht der Quelle beleuchtete Objekt erfasst. Hieraus lässt sich anhand der Verzerrung der Beleuchtung eine Höheninformation über das Objekt ermitteln. Dabei weist die Beleuchtung insbesondere eine vorgegebene Form wie beispielsweise eine Linie oder dergleichen auf. Diese wird durch die Topologie des Objekts verzerrt, so dass durch den ersten Sensor die verzerrte Beleuchtung erfasst wird und aus der Verzerrung eine Höheninformation ermittelt werden kann. Durch einen zweiten Sensor wird das von der Farblichtquelle erzeugte Farblicht, welches von dem Objekt reflektiert wird, erfasst. Hierdurch ist es möglich eine Farbinformation über das Objekt zu ermitteln. Aufgrund der Farbinformation ist es möglich das Objekt weiter zu charakterisieren und insbesondere ähnliche Objekte voneinander zu unterscheiden aufgrund einer unterschiedlichen Farbinformation. Handelt es sich bei dem Objekt insbesondere um eine Pflanze kann aufgrund der Farbinformation der Krankheitsstatus, der Ernährungsstatus und/oder die Reife von Früchten der Pflanze auf einfache Weise ermittelt werden.

Erfindungsgemäß handelt es sich bei der Quelle um einen Linienlaser. Somit wird das Objekt durch den Linienlaser linienweise beleuchtet, wobei die Linie des Linienlasers durch die Topologie des Objekts verzerrt wird und hieraus eine Höheninformation über das Objekt ermittelt werden kann entlang der Linie.

Vorzugsweise liegt die erste Wellenlänge im Bereich einer Bandlücke im Sonnenspektrum. Bandlücken entstehen durch Absorption von Sonnenlicht in der Atmosphäre. In den Bereichen dieser Bandlücke gelangt kein Sonnenlicht oder nur wenig Sonnenlicht auf die Erdoberfläche. Da im Bereich der Bandlücken kein oder nur wenig Sonnenlicht existiert, wirkt sich dieses nicht oder nur geringfügig störend auf die Objekterfassung aus.

Vorzugsweise beträgt die erste Wellenlänge 900nm bis 1000nm und liegt besonders bevorzugt im Bereich zwischen 920nm bis 960nm. Insbesondere beträgt die erste Wellenlänge 940nm.

Vorzugsweise wird die zweite Wellenlänge ausgewählt aus den Wellenlängenbereichen des Nahinfrarot (NIR), Rot, Grün und/ober Blau. Dabei beträgt die Wellenlänge des NIR 780nm bis 3µm, die von Rot 640nm bis 780nm, Grün 490nm bis 570nm und Blau 430nm bis 490nm. Insbesondere können weitere Farben hinzugenommen werden, wie beispielsweise Orange (600nm bis 640nm), Gelb (570nm bis 600nm), Violett (380nm bis 430nm) und nahes UV (200nm bis 380nm). Hierdurch ist es möglich eine spezifische Farbinformation von einem Objekt zu ermitteln.

Vorzugsweise sind mehrere Farblichtquellen vorgesehen, wobei alle Farblichtquellen insbesondere jeweils unterschiedliche Wellenlängen aufweisen und die Wellenlängen der Farblichtquellen ausgewählt sind aus NIR, Rot, Grün und/oder Blau. Insbesondere können die Wellenbereiche der einzelnen Farblichtquellen überlappend ausgebildet sein. Aufgrund des Vorsehens mehrerer Farblichtquellen mit unterschiedlichen Wellenlängen ist eine genauere Charakterisierung des Objekts möglich, besonders da eine detailliertere Aussage getroffen werden kann über den vorliegenden Krankheitsstatus, Ernährungsstatus und/oder Reifestatus der Früchte einer Pflanze oder alternativ hierzu bei der Charaktersierung und/oder Diskriminierung von ähnlichen, jedoch nicht gleichen Objekten wie beispielsweise Autos, Tieren und dergleichen.

Vorzugsweise handelt es sich bei der Farblichtquelle um eine oder mehrere LEDs. LED (Light Emitting Diodes) sind kostengünstig in einem breiten Emissionsspektrum zu erhalten. Insbesondere sind LEDs hell genug und können schnell geschaltet werden, so dass eine schnelle Erfassung von Objekten möglich ist. Hierzu kann es vorgesehen sein, dass je Wellenlänge bzw. Wellenlängenbereich genau eine LED vorgesehen ist oder alternativ hierzu kann für jede Wellenlänge bzw. jeden Wellenlängenbereich eine Vielzahl von LED vorgesehen sein, so dass sich deren Leuchtintensität kombiniert.

Vorzugsweise wird das von dem Objekt reflektierte Farblicht aus dem optischen Strahlengang der Quelle zur Erzeugung von Licht durch einen Strahlteiler heraus geleitet zum ersten bzw. zweiten Sensor. Hierbei ist offensichtlich, dass sofern das System lediglich einen ersten Sensor aufweist das reflektierte Farblicht durch den Strahlteiler zum ersten Sensor geleitet wird. Alternativ sofern das System einen ersten und zweiten Sensor aufweist, wird das reflektierte Farblicht zum zweiten Sensor durch einen Strahlteiler heraus geleitet. Alternativ oder zusätzlich zu einem Strahlteiler kann ein dichroitischer Spiegel vorgesehen sein, um wellenlängenselektiv das reflektierte Farblicht aus dem optischen Strahlengang der Quelle zur Erzeugung von Licht heraus zu leiten. Insbesondere kann durch den dichroitischen Spiegel, welcher besonders bevorzugt wellenlängenselektiv für die erste Wellenlänge abgestimmt ist, die erste Wellenlänge reflektiert, wohingegen die zweiten Wellenlängen transmittiert werden. Auch die umgekehrte Situation ist möglich, so dass die erste Wellenlänge transmittiert wird, wohingegen alle zweiten Wellenlängen reflektiert werden.

Vorzugsweise wird durch den ersten bzw. zweiten Sensor nur das Farblicht erfasst, wenn eine Farblichtquelle aktiv ist. Hierbei ist selbstverständlich, dass sofern das System ausschließlich einen ersten Sensor aufweist, welcher ebenfalls das reflektierte Farblicht erfasst, vom ersten Sensor nur das Farblicht erfasst wird, wenn eine Farblichtquelle aktiviert ist. Falls das System einen ersten und einen zweiten Sensor aufweist, wird von dem zweiten Sensor nur das reflektierte Farblicht erfasst, wenn eine Farblichtquelle aktiviert ist. Zusätzlich wird durch den ersten Sensor nur das Licht erfasst, wenn die Quelle aktiviert ist. Somit wird nur das reflektierte Farblicht erfasst, wenn eine Farblichtquelle tatsächlich aktiviert ist. Ein unbeabsichtigtes gegenseitiges Beeinflussen von Farblicht bzw. Licht wird somit verhindert. Insbesondere bei Vorsehen mehrerer Farblichtquellen werden nacheinander alle Farblichtquellen aktiviert, das reflektierte Farblicht wird erfasst und die Farblichtquelle wird deaktiviert, bevor eine weitere Farblichtquelle aktiviert wird. Hierdurch ist eine gegenseitige Beeinflussung der Farblichtquellen reduziert. Es können für jede Wellenlänge bzw. für jeden Wellenlängenbereich einer Farblichtquelle die Farbinformationen des Objekts unabhängig voneinander ermittelt werden und nachfolgend ausgewertet werden. Eine Vermischung aller Farbinformationen findet hierbei nicht statt. Kann beispielsweise eine Aussage über den Ernährungsstatus der Pflanze aufgrund des reflektierten grünen

Farblichts getroffen werden, so ist dies beispielsweise nicht möglich, wenn gleichzeitig das Objekt mit rotem Farblicht beleuchtet wird.

Erfindungsgemäß ist das Farblicht und das Licht der Quelle zumindest teilweise entlang desselben optischen Wegs auf das Objekt gerichtet und insbesondere auf dieselbe Stelle des Objekts gerichtet. Zusätzlich hierzu ist das Farblicht und das Licht der Quelle zumindest teilweise innerhalb einer gemeinsamen Ebene auf das Objekt gerichtet. Insbesondere können die Farblichtquellen beidseitig neben der Quelle zur Erzeugung von Licht vorgesehen werden und in dieselbe Richtung abstrahlen. Hierbei wären Farblicht und Licht der Quelle entlang des gesamten optischen Wegs auf das Objekt gerichtet und insbesondere innerhalb einer gemeinsamen Ebene. Alternativ hierzu können beispielsweise eine oder mehrere Farblichtquellen einseitig oder beidseitig neben einem Umlenkspiegel für das Licht angebracht werden und in Richtung des reflektierten Lichts auf das Objekt gerichtet sein. Hierbei ist der optische Weg des Farblichts und des Lichts ausgehend vom Umlenkspiegel derselbe bzw. innerhalb einer gemeinsamen Ebene. Hierdurch wird sichergestellt, dass zwischen dem Licht sowie dem Farblicht kein Versatz auf dem Objekt entsteht, welcher zu Fehlern in der Erfassung durch den ersten und/oder zweiten Sensor führen würde. Insbesondere ist es möglich, stets eine scharfe Abbildung des beleuchteten Bereichs des Objekts durch den ersten und/oder den zweiten Sensor zu erhalten. Zusätzlich ist durch diese Anordnung der Abstand zwischen Farblichtquelle und Objekt eindeutig bekannt. Hierdurch ist es insbesondere möglich eine Intensitätskorrektur des reflektierten Farblichts durchzuführen, die gerade diesen Abstand berücksichtigt. Dabei kann berücksichtigt werden, dass weiter entfernte Punkte auf dem Objekt eine geringere Intensität des Farblichts zum ersten bzw. zweiten Sensor zurückreflektieren als nähere Punkte auf dem Objekt.

Vorzugsweise ist das System bewegbar und/oder das Objekt bewegbar, wodurch das System relativ zum Objekt bewegt werden kann zur Erfassung des vollständigen Objekts. Hierbei erfolgt eine relative Bewegung zwischen System und Objekt, um nacheinander alle Teile des Objekts zu erfassen. Dabei kann insbesondere entweder eine kontinuierliche Bewegung von System und Objekt relativ zueinander erfolgen oder eine schrittweise Bewegung nach jeder Erfassung von Licht und/oder Farblicht.

Vorzugsweise handelt es sich bei dem ersten Sensor und/oder dem zweiten Sensor um einen CCD-Sensor. Vorzugsweise weist das System Spiegel zur Umlenkung des Farblichts oder des Lichts auf.

Vorzugsweise weist das System Linsen auf zur Abbildung des beleuchteten Objekts. Vorzugsweise weist das System Filter zur Wellenlängenselektion auf, wobei es sich insbesondere um einen Bandpass handelt vor dem ersten Sensor, welcher lediglich die Wellenlänge des Lichts durchlässt im Falle, dass das System einen ersten und einen zweiten Sensor aufweist. In diesem Fall kann ein weiterer Filter zur Wellenlängenselektion vorgesehen sein vor dem zweiten Sensor, ebenfalls ausgebildet als Bandpass, der nur Wellenlängen des Farblichts durchlässt.

Vorzugsweise handelt es sich bei dem Objekt um eine Pflanze, insbesondere um ein Ernteprodukt, ein Tier ein Auto oder dergleichen.

Weiterhin betrifft die vorliegende Erfindung ein Verfahren zur optischen Erfassung eines Objekts gemäß Anspruch 10.

Vorzugsweise wird ein Bild aufgenommen, ohne dass die Quelle zur Erzeugung von Licht sowie die Farblichtquelle aktiviert ist als Referenzbild. Hierdurch wird die Hintergrundbeleuchtung ermittelt, welche sodann bei der Auswertung zur Ermittlung der Höheninformation oder zur Ermittlung der Farbinformation berücksichtigt werden kann.

Vorzugsweise werden nacheinander mehrere Farblichtquellen insbesondere für NIR, Rot, Grün und Blau aktiviert und jeweils ein Bild aufgenommen. Somit wird nacheinander eine Farblichtquelle aktiviert, ein Bild aufgenommen und wieder deaktiviert.

Vorzugsweise werden die Schritte des Verfahrens als Zyklus mehrfach wiederholt zur vollständigen Erfassung des Objekts, wobei insbesondere die Quelle zur Erzeugung von Licht und/oder die Farblichtquelle relativ zum Objekt bewegt werden zur Erfassung des vollständigen Objekts. Hierzu kann das Objekt bewegt werden oder die Quelle zur Erzeugung von Licht und/oder die Farblichtquelle. Somit besteht ein Zyklus aus der Ermittlung der Höheninformation wie vorstehend beschrieben und nachfolgend der konsekutiven Ermittlung aller Farbinformationen. Hierbei kann selbstverständlich die Reihenfolge frei gewählt werden. Insbesondere wird in jedem Zyklus mindestens ein Referenzbild erzeugt. Durch die relative Bewegung zwischen Objekt und Quelle bzw. Farblichtquelle ist eine vollständige Erfassung des Objekts möglich. Hierbei kann eine solche relative Bewegung schrittweise erfolgen nach jedem Zyklus oder kontinuierlich.

Vorzugsweise werden innerhalb eines Zyklus mehrere Bilder für eine Farbe aufgenommen. Insbesondere ist dabei die Intensität des Farblichts bei den einzelnen Bildern einer Farbe unterschiedlich. So wird beispielsweise für jede Farbe zunächst ein erstes Bild aufgenommen mit der vollen Intensität der Farblichtquelle für die jeweilige Farbe und sodann ein zweites Bild für die selbe Farbe mit geringerer Intensität der Farblichtquelle. Hierdurch ist es möglich den Kontrast zu erhöhen insbesondere durch ein Übereinanderlegen der Bilder einer entsprechenden Farbe. Hierdurch kann ein High Dynamic Range (HDR-) Bild für jede der Farben erstellt werden.

Vorzugsweise ist das Verfahren weitergebildet anhand der Merkmale des Systems zur optischen Erfassung von Objekten wie vorstehend beschrieben und insbesondere gemäß den Ansprüchen 1 bis 9.

Nachfolgend wird die Erfindung anhand einer bevorzugten Ausführungsform unter Bezugnahme auf die beigefügten Zeichnungen näher erläutert.

Es zeigen:
- Fig. 1: eine erfindungsgemäße Ausführungsform des Systems zur optischen Erfassung von Objekten,
- Fig. 2: eine Detailansicht des in der Fig. 1 gezeigten Systems zur optischen Erfassung von Objekten,
- Fig. 3: ein Höhenprofil erfasst durch das System zur optischen Erfassung gemäß Fig. 1,
- Fig. 4: ein charakteristisches Reflektionsspektrum einer Pflanze,
- Fig. 5: ein Ablaufdiagramm des erfindungsgemäßen Verfahrens zur Erfassung eines Objekts und
- Fig. 6: ein Ablaufdiagramm eines weiteren erfindungsgemäßen Verfahrens zur Erfassung eines Objekts.

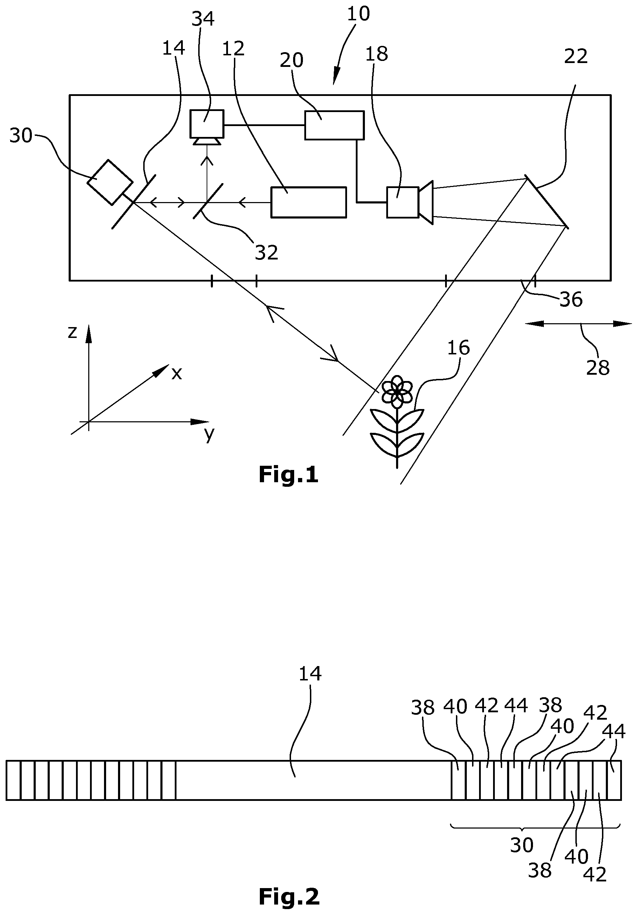

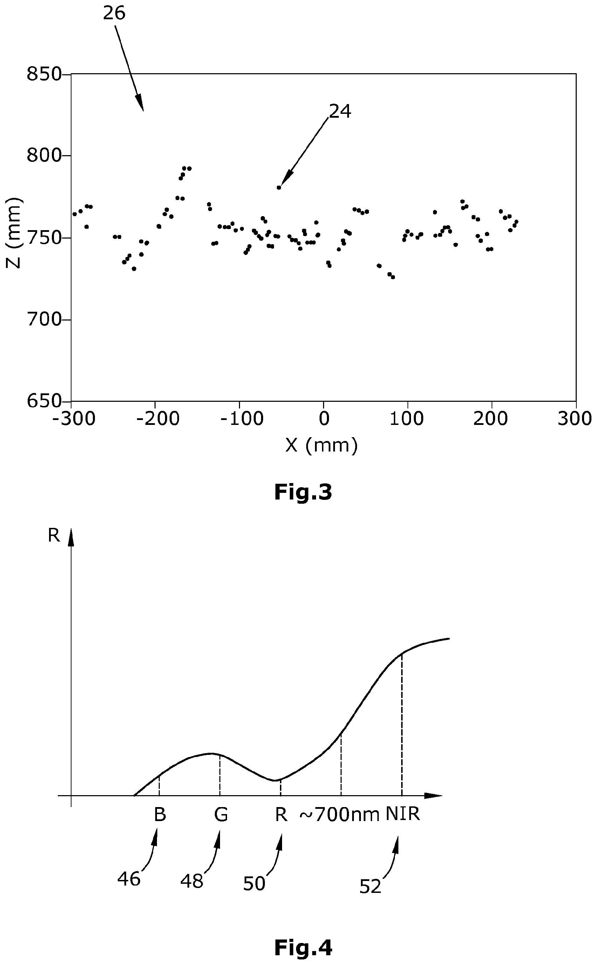

Das erfindungsgemäße System zur optischen Erfassung von Objekten, gezeigt in Fig. 1, weist ein Gehäuse 10 auf. Innerhalb des Gehäuses 10 ist eine Quelle 12 zur Erzeugung von Licht angeordnet, wobei es sich beispielsweise um einen Linienlaser handelt. Das Licht des Linienlasers 12 beleuchtet über einen Spiegel 14 ein Objekt 16, dargestellt als Pflanze. Durch die Topologie der Pflanze 16 erfolgt eine Verzerrung der linienförmigen Beleuchtung des Linienlasers 12. Durch einen ersten Sensor 18, ebenfalls angeordnet innerhalb des Gehäuses 10 wird über einen Spiegel 22 das Objekt 16 und insbesondere die verzerrte linienförmige Beleuchtung des Objekts 16 erfasst. Der erste Sensor 18 ist mit einer Auswerteeinrichtung 20 verbunden, wobei durch die Auswerteeinrichtung anhand der Verzerrung der linienförmigen Beleuchtung durch das Objekt eine Höheninformation über das Objekt entlang der linienförmigen Beleuchtung ermittelt werden kann. Ein typisches Höhenprofil ist in Fig. 3 gezeigt. Entlang der Linie des Linienlasers 12 wird eine Vielzahl von Datenpunkten 24 ermittelt, welche zusammen ein Höhenprofil 26 ergeben. Dabei ist das gesamte Gehäuse 10 des Systems entlang der Pfeile 28 in y-Richtung bewegbar, so dass nacheinander die gesamte Topologie des Objekts 16 erfasst werden kann durch eine Vielzahl von Höhenprofilen 26.

Zusätzlich weist das System mehrere Farblichtquellen 30 auf, die als LEDs ausgebildet sind und rotes, grünes, blaues und nah infrarotes Licht erzeugen können. Über die Farblichtquellen 30 wird das Objekt 16 beleuchtet. Dabei erfolgt die Beleuchtung durch die Farblichtquellen 30 in einer identischen Ebene zu der Beleuchtung mit dem Linienlaser 12. In der Fig. 1 ist dabei die gemeinsame Ebene senkrecht zur y/z-Ebene. Das von dem Objekt 16 reflektierte Farblicht gelangt über den Spiegel 14 und einen dichroitischen Spiegel 32 zu einem zweiten Sensor 34. Dabei wird vom dichroitischen Spiegel 32 Licht in der Wellenlänge des Linienlasers 12 transmittiert und alle weiteren Wellenlängen reflektiert. Vom zweiten Sensor 34 wird das reflektierte Farblicht erfasst und durch die Auswerteeinheit 20 wird eine Farbinformation über das Objekt ermittelt.

Erster Sensor 18 und zweiter Sensor 34 sind beispielsweise als CCD Kameras ausgebildet.

Um eine Beeinträchtigung des ersten Sensors 18 durch Sonnenlicht zu verhindern, weist das Gehäuse 10 einen Filtereingang 36 auf, der als Bandpass ausgebildet ist und ausschließlich Licht der Wellenlänge des Linienlasers 12 transmittiert. Insbesondere ist dabei die Wellenlänge des Linienlasers 12 ausgewählt, um einer Bandlücke im Sonnenspektrum zu entsprechen, so dass eine geringe oder keine Beeinflussung durch Sonnenlicht erfolgt.

Die Farblichtquellen 30 sind dabei beidseitig neben dem Spiegel 14 angeordnet (wie in Fig. 2 dargestellt). Eine erste Farblichtquelle bestehend aus drei Rotlicht-LEDs 38 auf der einen Seite und ebenfalls drei Rotlicht-LEDs auf der anderen Seite sind alternierend angeordnet mit Grünlicht-LEDs 40, Blaulicht-LEDs 42 und LEDs 44 zur Erzeugung von nahinfrarotem Licht. Dabei ist der Aufbau links und rechts vom Spiegel 14 insbesondere symmetrisch ausgebildet.

Durch die Farbinformationen, welche mittels der Farblichtquelle 30 und dem zweiten Sensor 34 ermittelt wird, ist es möglich eine genaue Charakterisierung des Objekts 16 durchzuführen und insbesondere ähnliche, jedoch nicht exakt gleiche Objekte 16 voneinander zu unterscheiden. Handelt es sich bei dem Objekt beispielsweise um eine Pflanze, ist es möglich, über die Farbinformation einen Ernährungsstatus der Pflanze zu ermitteln und/oder Krankheiten bzw. den Krankheitsstatus der Pflanze zu ermitteln. Auch kann aufgrund der Farbinformation die Reife der Früchte der Pflanze ermittelt werden. Insbesondere liefert die Farbinformation eine Unterscheidungsmöglichkeit, ob es sich tatsächlich um eine Pflanze handelt. In Fig. 4 ist hierzu ein charakteristisches Reflektionsspektrum einer Pflanze dargestellt mit einem geringen Blauanteil 46, einem hohen Grünanteil 48, einem geringen Rotanteil 50 und einem charakteristischen steilen Anstieg im Nahinfraroten 52. Insbesondere über den Unterschied zwischen dem roten Bereich 50 und dem nahinfraroten Bereich 52 kann aus der Farbinformation erkannt werden, ob es sich tatsächlich um eine Pflanze oder um ein anderes nicht pflanzliches Objekt handelt.

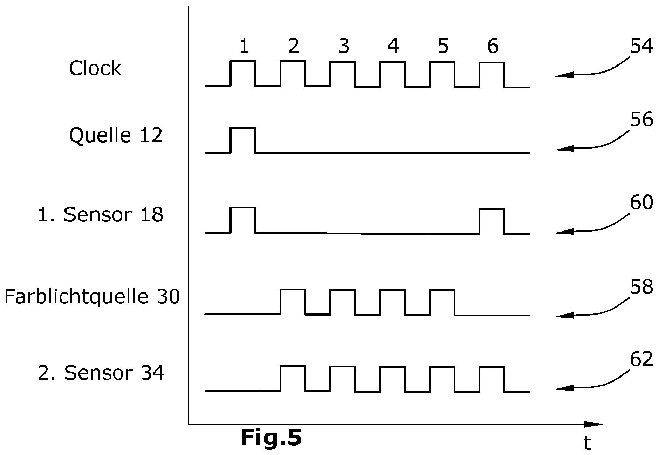

In Fig. 5 ist ein Zyklus des erfindungsgemäßen Verfahrens zur optischen Erfassung von Objekten dargestellt. Der Zyklus umfasst sechs Pulse. Jeder Puls hat eine Länge von 0,05 bis 1µs. Die Frequenz der Zyklen beträgt 10 bis 500Hz. In der ersten Zeile 54 ist die Pulsfolge eines Zyklus erzeugt durch einen Pulsgeber dargestellt. In der zweiten Zeile 56 ist die Aktivierung des Linienlasers 12 dargestellt. Dieser wird beim ersten Puls aktiviert. Gleichzeitig wird gemäß der zweiten Zeile 56 ein Bild durch den ersten Sensor 18 aufgenommen. Durch den zweiten Sensor 34 wird beim ersten Puls kein Bild aufgenommen. Auch die Farblichtquellen 30 bleiben deaktiviert. Vom zweiten bis zum fünften Puls werden nacheinander alle Farblichtquellen aktiviert und gleichzeitig ein Bild durch den zweiten Sensor 34 aufgenommen entsprechend den dritten und vierten Zeile 58, 62 der Fig. 5. So wird beispielsweise zum zweiten Puls die Rotlicht-LED 38 aktiviert, bei einem dritten Puls die Grünlicht-LED 40, beim vierten Puls die Blaulicht-LED 42 und beim fünften Puls die Nahinfrarot-LED 44. Aus diesen Bildern lässt sich die Farbinformation durch die Auswerteeinheit 20 ermitteln. Beim sechsten Puls ist weder die Farblichtquelle noch der Linienlaser 12 aktiviert und durch den ersten Sensor 18 und den zweiten Sensor 34 wird ein Bild aufgenommen als Referenzbild.

Nach dem Durchlaufen eines vollständigen Zyklus oder kontinuierlich wird das System relativ zum Objekt 16 entsprechend der Pfeile 28 verschoben. Hierdurch wird das Objekt 16 vollständig erfasst. Insbesondere werden vollständige Farbinformationen des Objekts 16 erfasst, um das Objekt 16 genau charakterisieren zu können.

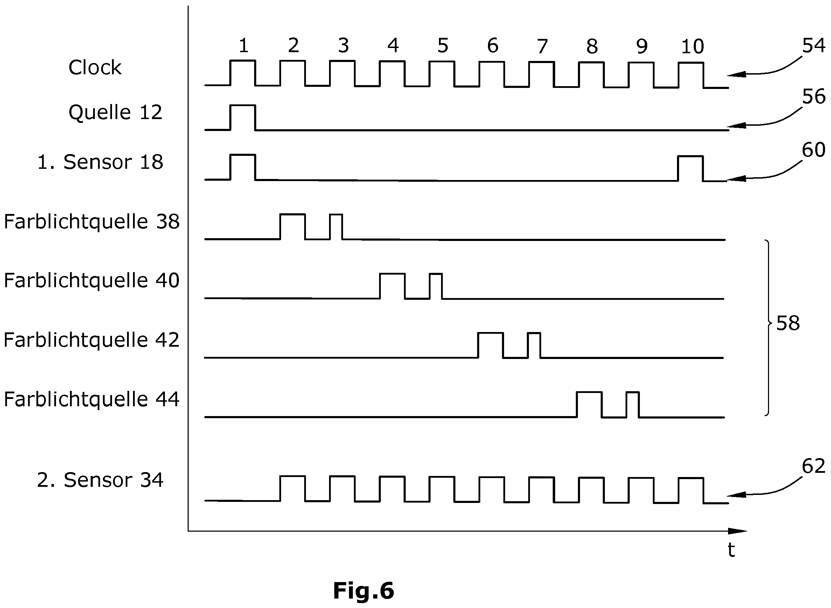

In Fig. 6 ist ein Zyklus eines weiteren erfindungsgemäßen Verfahrens zur optischen Erfassung von Objekten dargestellt. Hierbei werden für jede Farbe zwei Bilder aufgenommen, also jede Farblichtquelle 38, 40, 42, 44 in einem Zyklus zweimal aktiviert. Entsprechend erfolgt eine Aktivierung des zweiten Sensors 34. Dabei wird für jede Farbe zunächst ein Bild mit voller Intensität des Farblichts erstellt und sodann bei der zweiten Aktivierung der Farblichtquelle 38, 40, 42, 44 durch eine verkürzte Aktivierung ein Bild mit geringerer Intensität des Farblichts erstellt. Dies ist in den entsprechenden Zeilen der Fig. 6 dargestellt. Der sonstige Ablauf des Verfahrens entspricht dem Verfahren der Fig. 5.

## Patentansprüche

1. System zur optischen Erfassung von Objekten, mittels Lasertriangulation mit
einer Quelle (12) zur Erzeugung von Licht einer ersten Wellenlänge, wobei die Quelle ausgebildet ist, um durch das Licht das Objekt (16) zu beleuchten, wobei die Beleuchtung durch das Objekt (16) verzerrt wird,
mindestens einer Farblichtquelle (30) zur Erzeugung von Farblicht einer zweiten Wellenlänge, wobei die Farblichtquelle ausgebildet ist, um durch das Farblicht der Farblichtquelle (30) das Objekt (16) zu beleuchten,
wobei die erste Wellenlänge verschieden ist von der zweiten Wellenlänge,
einem ersten Sensor (18) zur Erfassung des durch das Licht der Quelle (12) beleuchteten Objekts (16) zur Ermittlung einer Höheninformation des Objekts und
einem zweiten Sensor (34) zur Erfassung des von der Farblichtquelle (30) erzeugten und durch das Objekt (16) reflektierten Farblichts zur Ermittlung einer Farbinformation über das Objekt,
wobei es sich bei der Quelle (12) um einen Linienlaser handelt,
wobei die Beleuchtung durch die Farblichtquelle (30) in einer identischen Ebene zu der Beleuchtung mit dem Linienlaser (12) erfolgt.

2. System nach Anspruch 1, **dadurch gekennzeichnet, dass** es sich bei der Farblichtquelle (30) um eine oder mehrere LEDs handelt.

3. System nach Anspruch 1 oder 2, **dadurch gekennzeichnet, dass** die erste Wellenlänge im Bereich einer Absorptionsbande im Sonnenspektrum liegt und insbesondere im Bereich von 900nm bis 1000nm, vorzugsweise im Bereich von 920nm bis 960nm liegt und besonders bevorzugt 940nm beträgt.

4. System nach einem der Ansprüche 1 bis 3, **dadurch gekennzeichnet, dass** die zweite Wellenlänge einer der Farben NIR, Rot, Grün, Blau ist.

5. System nach einem der Ansprüche 1 bis 4, **dadurch gekennzeichnet, dass** mehrere Farblichtquellen (38, 40, 42, 44) vorgesehen sind und alle Farblichtquellen (38, 40, 42, 44) jeweils unterschiedliche Wellenlängen aufweisen und die Wellenlänge aller Farblichtquellen (38, 40, 42, 44) ausgewählt sind aus der Liste NIR, Rot, Grün, Blau.

6. System nach einem der Ansprüche 1 bis 5, **dadurch gekennzeichnet, dass** ein Strahlteiler oder ein dichroitischer Spiegel (32) im optischen Strahlengang der Quelle vorgesehen ist, so dass das reflektierte Farblicht zum ersten bzw. zweiten Sensor (18, 34) ablenkbar ist.

7. System nach einem der Ansprüche 1 bis 6, **dadurch gekennzeichnet, dass** der erste bzw. zweite Sensor (18, 34) ausgebildet ist, so dass durch den ersten bzw. zweiten Sensor nur das reflektierte Farblicht erfassbar ist bei aktivierter Farblichtquelle (30) und dass der erste Sensor (18) ausgebildet ist, so dass durch den ersten Sensor nur das Licht erfassbar ist bei aktivierter Quelle (12).

8. System nach einem der Ansprüche 1 bis 7, **dadurch gekennzeichnet, dass** das Farblicht und das Licht der Quelle zumindest teilweise entlang desselben optischen Wegs auf das Objekt (16) gerichtet ist.

9. System nach einem der Ansprüche 1 bis 8, **dadurch gekennzeichnet, dass** das System relativ zum Objekt (16) bewegbar ist zur Erfassung des vollständigen Objekts (16), wobei das System bewegbar ist und/oder das Objekt (16) bewegbar ist.

10. Verfahren zur optischen Erfassung eines Objekts mittels Lasertriangulation, bei welchem
a. eine Quelle (12) zur Erzeugung von Licht einer ersten Wellenlänge aktiviert wird, um das Objekt (16) zu beleuchten, wobei die Beleuchtung durch das Objekt (16) verzerrt wird,
b. ein Bild aufgenommen wird und aus der Verzerrung eine Höheninformation über das Objekt (16) ermittelt wird,
c. die Quelle (12) zur Erzeugung von Licht deaktiviert wird,
d. eine Farblichtquelle (30) zur Erzeugung von Farblicht einer zweiten Wellenlänge aktiviert wird, wobei durch das Farblicht der Farblichtquelle (30) das Objekt (16) beleuchtet wird,
e. ein Bild des reflektierten Farblichts aufgenommen wird und aus dem reflektierten Farblicht eine Farbinformation über das Objekt (16) ermittelt wird und
f. die Farblichtquelle (30) deaktiviert wird,
wobei die erste Wellenlänge verschieden ist von der zweiten Wellenlänge und die zweite Wellenlänge ausgewählt wird aus NIR, Rot, Grün, Gelb oder Blau,
wobei es sich bei der Quelle (12) um einen Linienlaser handelt,
wobei die Beleuchtung durch die Farblichtquelle (30) in einer identischen Ebene zu der Beleuchtung mit dem Linienlaser (12) erfolgt.

11. Verfahren nach Anspruch 10, bei welchem ein Bild als Referenzbild aufgenommen wird, ohne dass die Quelle (12) zur Erzeugung von Licht oder die Farblichtquelle (30) aktiviert ist.

12. Verfahren nach Anspruch 10 oder 11, bei welchem nacheinander mehrere Farblichtquellen (38, 40, 42, 44) insbesondere für NIR, Rot, Grün, Blau aktiviert werden und jeweils ein Bild aufgenommen wird.

13. Verfahren nach einem der Ansprüche 10 bis 12, bei welchem als Zyklus alle Schritte mehrfach wiederholt werden zur vollständigen Erfassung des Objekts (16), wobei insbesondere die Quelle (12) zur Erzeugung von Licht und/oder die Farblichtquelle (30) relativ zum Objekt (16) bewegt wird zur Erfassung des vollständigen Objekts (16).

14. Verfahren nach einem der Ansprüche 10 bis 13, bei welchem die Intensität des Farblichts veränderbar ist und insbesondere innerhalb eines Zyklus ein erstes Bild mit Farblicht einer bestimmten Wellenlänge aufgenommen wird und ein zweites Bild mit Farblicht derselben Wellenlänge aufgenommen wird, wobei sich die Intensität des Farblichts für das erste Bild und das zweite Bild unterscheidet.

## Claims

1. System for the optical detection of objects by means of laser triangulation, comprising
a source (12) for generating light of a first wavelength, wherein the source is configured to illuminate the object (16) by the light, wherein the illumination is distorted by the object (16),
at least one color light source (30) for generating color light of a second wavelength, wherein the color light source is configured to illuminate the object (16) by the color light of the color light source (30),
wherein the first wavelength is different from the second wavelength,
a first sensor (18) for detecting the object (16) illuminated by the light of the source (12) for determining height information about the object, and
a second sensor (34) for detecting the color light generated by the color light source (30) and reflected by the object (16) for determining color information about the object,
wherein the source (12) is a line laser,
wherein the illumination by the color light source (30) is effected in a plane identical to the illumination by the line laser (12).

2. System of claim 1, **characterized in that** the source (12) is a line laser and the color light source (30) preferably is one or a plurality of LEDs.

3. System of claim 1 or 2, **characterized in that** the first wavelength is in the range of a band gap in the solar spectrum and in particular in the range from 900 nm to 1000 nm, preferably in the range from 930 nm to 960 nm, and particularly preferred is 940 nm.

4. System of one of claims 1 to 3, **characterized in that** the second wavelength is selected from NIR, red, green, blue.

5. System of one of claims 1 to 4, **characterized in that** a plurality of color light sources (38, 40, 42, 44) is provided, and all color light sources (38, 40, 42, 44) have different wavelengths, respectively, and the wavelength of all color light sources (38, 40., 42, 44) is selected in particular from the list NIR, red, green, blue.

6. System of one of claims 1 to 5, **characterized in that** a beam splitter or a dichroic mirror (32) is provided in the optical beam path of the source, so that the reflected color light can be diverted to the first or the second sensor (18, 34).

7. System of one of claims 1 to 6, **characterized in that** the first or the second sensor (18, 34) is configured such that the first or second sensor only detects the reflected color light when a color light source (30) is activated, and that the first sensor (18) is configured such that the first sensor only detects the light when the source (12) is activated.

8. System of one of claims 1 to 7, **characterized in that** the color light and the light of the source are directed to the object (16) at least partly along the same optical path.

9. System of one of claims 1 to 8, **characterized in that** the system is movable relative to the object (16) to detect all of the object (16), wherein the system is movable and/or the object (16) is movable.

10. Method for the optical detection of an object by means of laser triangulation, in which
a. a source (12) for generating light of a first wavelength is activated to illuminate the object (16), wherein the illumination is distorted by the object (16),
b. an image is captured and elevation information about the object (16) is determined from the distortion,
c. the source (12) for generating ligh is deactivated,
d. a color light source (30) for generating color light of a second wavelength is activated, wherein the object (16) is illuminated by the color light of the color light source (30),
e. an image of the reflected color light is captured and color information about the object is determined from the reflected color light, and
f. the color light source (30) is deactivated,
wherein the first wavelength is different from the second wavelength and the second wavelength is selected in particular from NIR, red, green, yellow or blue,
wherein the source (12) is a line laser,
wherein the illumination by the color light source (30) is effected in a plane identical to the illumination by the line laser (12).

11. Method of claim 10, in which an image is captured as a reference image without the source (12) for generating light or the color light source (30) being activated.

12. Method of claim 10 or 11, in which a plurality of c or light sources (38, 40, 42, 44) in particular for NIR, red, green, blue are activated subsequently and one image is captured, respectively.

13. Method of one of claims 10 to 12, in which all steps are repeated several times as a cycle for a complete detection of the object (16), wherein in particular the source (12) for generating light and/or the color light source (30) are moved relative to the object (16) to detect the object (16) in its entirety.

14. Method of one of claims 10 to 13, in which the intensity of the color light is variable and, in particular within a cycle, a first image is captured with color light of a defined wavelength and a second image is captured with color light of the same wavelength, wherein the intensity of the color light differs for the first image and the second image.

## Revendications

1. Système de détection optique d'objets, par triangulation laser avec
une source (12) pour générer de la lumière d'une première longueur d'onde, où la source est conçue pour éclairer l'objet (16) par la lumière, où l'éclairage est distorsionné par l'objet (16),
au moins une source de lumière colorée (30) pour générer de la lumière colorée d'une deuxième longueur d'onde, où la source de lumière colorée est conçue pour éclairer l'objet (16) par la lumière colorée de la source de lumière colorée (30),
dans lequel la première longueur d'onde est différente de la deuxième longueur d'onde,
un premier capteur (18) destiné à détecter l'objet (16) éclairé par la lumière de la source (12) pour déterminer une information de hauteur de l'objet, et
un deuxième capteur (34) destiné à détecter la lumière colorée générée par la source de lumière colorée (30) et réfléchie par l'objet (16) pour déterminer une information de couleur relative à l'objet,
dans lequel il s'agit pour la source (12) d'un laser linéaire,
dans lequel l'éclairage par la source de lumière colorée (30) a lieu dans un plan identique à l'éclairage par le laser linéaire (12).

2. Système selon la revendication 1, **caractérisé par le fait qu'**il s'agit pour la source de lumière colorée (30) d'une ou plusieurs LED.

3. Système selon la revendication 1 ou 2, **caractérisé par le fait que** la première longueur d'onde se situe dans la plage d'une bande d'absorption dans le spectre solaire et en particulier dans la plage de 900nm à 1000nm, de préférence dans la plage de 920nm à 960nm et de manière particulièrement préférée de 940nm.

4. Système selon l'une des revendications 1 à 3, **caractérisé par le fait que** la deuxième longueur d'onde est l'une des couleurs NIR, rouge, vert, bleu.

5. Système selon l'une des revendications 1 à 4, **caractérisé par le fait qu'**il est prévu plusieurs sources de lumière colorée (38, 40, 42, 44) et que toutes les sources de lumière colorée (38, 40, 42, 44) présentent, chacune, des longueurs d'onde différentes et que la longueur d'onde des sources de lumière colorée (38, 40, 42, 44) est sélectionnée dans la liste de NIR, rouge, vert, bleu.

6. Système selon l'une des revendications 1 à 5, **caractérisé par le fait qu'**il est prévu un diviseur de faisceau ou un miroir dichroïque (32) dans le trajet de faisceau optique de la source, de sorte que la lumière colorée réfléchie puisse être déviée vers le premier ou le deuxième capteur (18, 34).

7. Système selon l'une des revendications 1 à 6, **caractérisé par le fait que** le premier ou le deuxième capteur (18, 34) est conçu de sorte que seule la lumière colorée réfléchie puisse être détectée par le premier ou le deuxième capteur lorsque la source de lumière colorée (30) est activée et que le premier capteur (18) est conçu de sorte que seule la lumière puisse être détectée par le premier capteur lorsque la source (12) est activée.

8. Système selon l'une des revendications 1 à 7, **caractérisé par le fait que** la lumière colorée et la lumière de la source sont orientées vers l'objet (16) au moins partiellement le long du même trajet optique.

9. Système selon l'une des revendications 1 à 8, **caractérisé par le fait que** le système est déplaçable par rapport à l'objet (16) pour détecter l'objet complet (16), dans lequel le système est déplaçable et/ou l'objet (16) est déplaçable.

10. Procédé de détection optique d'un objet par triangulation laser, dans lequel
a. une source (12) destinée à générer de la lumière d'une première longueur d'onde est activée pour éclairer l'objet (16), où l'éclairage est distorsionné par l'objet (16),
b. une image est prise et à partir de la distorsion est déterminée une information de hauteur relative à l'objet (16),
c. la source (12) destinée à générer de la lumière est désactivée,
d. une source de lumière colorée (30) destinée à générer de la lumière colorée d'une deuxième longueur d'onde est activée, où l'objet (16) est éclairé par la lumière colorée de la source de lumière colorée (30),
e. une image de la lumière colorée réfléchie est prise et à partir de la lumière colorée réfléchie est déterminée une information de couleur relative à l'objet (16), et
f. la source de lumière colorée (30) est désactivée,
dans lequel la première longueur d'onde est différente de la deuxième longueur d'onde et la deuxième longueur d'onde est choisie parmi NIR, rouge, vert, jaune ou bleu,
dans lequel la source (12) est un laser linéaire,
dans lequel l'éclairage par la source de lumière colorée (30) a lieu dans un plan identique à l'éclairage par le laser linéaire (12).

11. Procédé selon la revendication 10, dans lequel une image est prise comme image de référence sans que la source (12) destinée à générer de la lumière ou la source de lumière colorée (30) ne soit activée.

12. Procédé selon la revendication 10 ou 11, dans lequel plusieurs sources de lumière colorée (38, 40, 42, 44), notamment pour NIR, rouge, vert, bleu, sont activées successivement et il est chaque fois pris une image.

13. Procédé selon l'une des revendications 10 à 12, dans lequel, comme cycle, toutes les étapes sont répétées plusieurs fois pour la détection complète de l'objet (16), en particulier la source (12) destinée à générer de la lumière et/ou la source de lumière colorée (30) étant déplacée par rapport à l'objet (16) pour la détection de l'objet complet (16).

14. Procédé selon l'une des revendications 10 à 13, dans lequel l'intensité de la lumière colorée peut être modifiée et en particulier, dans un cycle, une première image est prise à l'aide de lumière colorée d'une longueur d'onde déterminée et une deuxième image est prise à l'aide de lumière colorée de la même longueur d'onde, où l'intensité de la lumière colorée pour la première image et la deuxième image est différente.
